# FASCICULE DE BREVET EUROPEEN

(11) **EP 0 471 597 B1**
(45) Date de publication et mention de la délivrance du brevet: **21.06.1995**
(21) Numéro de dépôt: 91402018.5
(22) Date de dépôt: 19.07.1991
(51) Int. Cl.: C08L 5/00, C04B 30/02, C02F 1/52

(54) **Composition comportant un polysaccharide obtenu par fermentation microbienne**
Zusammensetzung, die ein durch mikrobielle Fermentierung hergestelltes Polysaccharid enthält
Composition comprising a polysaccharide obtained by microbial fermentation

(30) Priorité: 30.07.1990 FR 9009669
(43) Date de publication de la demande: 19.02.1992
(73) Titulaire: RHONE-POULENC CHIMIE, 92408 Courbevoie Cédex (FR)
(72) Inventeur: Knipper, Magali, F-75019 Paris (FR); David, Claire, F-75014 Paris (FR); Besnard, Marie-Madeleine, F-92160 Antony (FR)
(74) Mandataire: Seugnet, Jean Louis

(56) Documents cités:
- EP-A- 0 013 217
- EP-A- 0 291 646
- WO-A-91/00176
- DE-B- 1 297 048
- GB-A- 2 058 812
- US-A- 3 406 114

## Description

La présente invention concerne une composition comportant un polysaccharide obtenu par fermentation microbienne et un polysaccharide cationique, ainsi que des applications de ladite composition.

Il est connu d'utiliser de la gomme Xanthane, éventuellement additionnée d'autres gommes du type gomme de caroube, agar-agar, guaranates ou alginates, en association avec un agent flocculant à base d'un métal du groupe du fer et/ou de l'aluminium (EP-A-13217).

Des polysaccharides dont le motif de base contient du glucose et du rhamnose sont bien connus. Ils sont habituellement obtenus par fermentation d'une source carbonée au moyen d'un microorganisme.

De tels polysaccharides et leurs procédés de préparation ont été décrit notamment dans les demandes de brevets européen N° 77680, N° 339 445 ou la demande de brevet français N° 88 09999.

Les polysaccharides obtenus par fermentation d'un milieu nutritif contenant un sucre au moyen d'un microorganisme Alcaligenes ou Pseudomonas peuvent être utilisés pour la flocculation de particules finement divisées suspendues en milieu aqueux (US-A-3.406.114).

La présente invention concerne une composition caractérisée en ce qu'elle comporte :
. au moins un polysaccharide (A) dont le motif de base contient du glucose et du rhamnose en quantités relatives correspondant à 1 à 6 moles (de préférence 1 à 4 moles) de glucose pour une mole de rhamnose, ledit polysaccharide étant préparé par fermentation d'un milieu nutritif comportant au moins une source carbonée au moyen d'un microorganisme Alcaligenes ou Pseudomonas;
. ainsi qu'au moins un polysaccharide cationique choisi parmi les dérivés cationiques d'amidon, de galactomannane ou de guar;
selon un rapport poids de polysaccharide (A)/poids de polysaccharide cationique compris entre 5/95 et 95/5, de préférence compris entre 30/70 et 70/30.

En outre, le motif de base dudit polysaccharide (A) peut contenir des restes d'autres acides organiques, tels que des restes pyruviles ou acétyles selon une quantité allant de 1 mole (de préférence 0,5 mole) d'acide organique pour 1 mole de glucose à 0 mole d'acide organique pour 6 moles de glucose, ainsi que d'autres sucres comme l'acide glucuronique, le galactose, le mannose, l'arabinose, le fucose ou le ribose selon une quantité allant de 2 moles (de preférence 1 mole) de sucre pour 1 mole de glucose à 0 mole de sucre pour 6 moles de glucose.

Les polysaccharides (A) sont obtenus par fermentation d'au moins une source carbonée comprise dans un milieu nutritif, au moyen d'un microorganisme Pseudomonas, de préférence Pseudomonas Paucimobilis et plus préférentiellement les souches I - 886 déposée à la CNCM et la souche DSM 4429, au genre Alcaligènes, de préférence la souche Alcaligènes ATCC 31961. Les milieux nutritif comportant la source carbonée, la nature de la souche carbonée ainsi que les procédés de préparation de tels polysaccharides sont décrit dans la littérature. On peut notamment se référer aux demandes de brevet européen N° 77680 et N° 339445 ainsi qu'à la demande de brevet français N° 88 09999, qui décrivent également des polysaccharides obtenus par fermentation microbienne

Les dérivés cationiques des amidons et des galactomannanes, en particulier le guar cationique sont bien connus de l'homme de l'art et sont habituellement disponibles dans le commerce. Ils sont généralement préparés par éthérification ou estérification des groupements hydroxyles libres des sucres formant le polysaccharide non cationique dont ils derivent, au moyen de composés organiques ammonium quaternaires, tel le chlorure de 2,3 époxyméthyl ammonium.

Les polysaccharides cationiques entrant dans le cadre de la présente invention ont un poids moléculaire qui est habituellement supérieur à 200 000, généralement compris entre 200 000 et 3 000 000.

La composition selon l'invention peut comprendre, outre les polysaccharides décrits ci-dessus, un additif floculant.

Dans le cadre de la présente invention, on entend par additif floculant un composé qui permet ou favorise la formation de flocs constitués lors de la mise en solution aqueuse desdits polysaccharides obtenus par fermentation microbienne et desdits polysaccharides naturels ou de leurs dérivés.

A titre d'additifs floculants, on peut citer les composés organiques ammonium quaternaire, l'ammoniaque, les polymères ou copolymères organiques anioniques de synthèse et leurs sels, ainsi que des composés à base d'un métal du groupe du fer et/ou de l'aluminium, tels que sulfate, chlorure, hydroxychlorure ou chlorosulfate d'aluminium et/ou de fer.

Les composés ammonium quaternaires peuvent être plus particulièrement les sels de n alkyltriméthylammonium, de n dialkyldiméthylammonium, de n alkyldiméthylammonium, de n alkylpyridinium et de benzalkonium. Dans ces composés, la chaîne alkyle peut comporter de 1 à 30, de préférence de 8 à 24 atomes de carbone.

Lesdits polymères ou copolymères organiques anioniques peuvent être à base d'acide acrylique ou d'acide méthacrylique, comme l'acide polyacrylique et l'acide polyméthacrylique.

Le poids en agent floculant compris dans la composition selon l'invention est généralement compris entre 0,0001 et 0,03 % du poids total des polysaccharides obtenu par fermentation microbienne et desdits polysaccharides naturels ou de leurs dérivés.

La composition selon l'invention peut être préparée par simple mélange desdits polysaccharides mis en poudre, ou par mélange d'un desdits polysaccharides en poudre dans une solution aqueuse comportant l'autre polysaccharide.

Préférentiellement, on peut également mettre en solution ledit polysaccharide obtenu par fermentation microbienne et ledit polysaccharide naturel ou un de ses dérivés séparément, puis mélanger sous agitation les deux solutions obtenues. L'agitation doit être suffisante pour obtenir une solution homogène. L'additif floculant peut être ajouté à tout moment dans l'une ou l'autre desdites solutions, mais, de préférence, dans ladite solution homogène. Cette dernière peut comporter de 0,001 à 0,3 % en poids, de préférence de 0,002 à 0,6 % en poids, de la composition selon l'invention.

La présente invention concerne également un procédé pour précipiter dans un milieu aqueux une dispersion de particules solides au moyen d'une composition telle que décrite ci-dessus.

De telles particules peuvent être de nature organiques ou minérales et de dimensions diverses. Leur concentration dans le milieu aqueux où elles sont dispersées est variable. Il est à noter ici qu'un tel effet précipitant de la composition selon l'invention est surprenant dans la mesure où il est connu que lesdits polysaccharides obtenus par fermentation microbienne et les polysaccharides naturels ou leurs dérivés sont habituellement utilisés comme agent de suspension.

Il importe toutefois pour obtenir une bonne précipitation que la concentration de la composition selon l'invention dans ledit milieu aqueux, soit comprise entre 0,001 et 0,5 %, de préférence de 0,005 à 0,2 % en poids.

Au cours dudit procédé, la composition selon l'invention peut être mélangée à ladite dispersion, par exemple sous la forme d'un solution homogène, telle que décrite ci-dessus.

Un procédé tel que décrit ci-dessus peut notamment être utilisé pour traiter des eaux usées comportant des particules solides en suspension et que l'on fait floculer, puis précipiter.

Ce procédé peut également servir à la préparation d'articles isolants à base de fibres minérales comportant la composition selon l'invention.

De tels articles peuvent se présenter sous la forme de panneaux ou de plaques pouvant supporter de très hautes températures. Ils peuvent donc être utilisés comme protection contre le feu, comme isolant réfractaire dans les fours thermiques et les chambres de combustion ou même comme isolant acoustique.

Outre lesdites fibres minérales et la composition selon l'invention, ces articles isolants peuvent également comporter au moins une charge minérale.

A titre de fibres minérales, on peut citer les fibres de bore, les fibres de carbone, les fibres de verre et les fibres céramiques, telles que les fibres d'alumine, de silice-alumine et de silice-alumine modifiées par d'autres oxydes tels que les oxydes de chrome, de bore, de zirconium, de calcium, de magnésium, les fibres d'oxyde de titane, de carbure de silicium, de nitrure de silicium, de carbonitrure, de nitrure de bore, ainsi que des laines minérales, comme la laine de diabase, la laine de roche ou la laine de laitier. Ces fibres ont favorablement une longueur de 0,1 à 50 mm et un diamètre de 1 à 20 microns.

Les charges minérales entrant dans la composition de l'article isolant peuvent être constituées par des silices telles les silices colloïdales, les alumines, la bentonite, la magnésie, le carbonate de calcium, le kaolin ou les silicates d'aluminium.

Les articles isolants selon l'invention peuvent être obtenus à partir d'une dispersion aqueuse de fibres minérales, pouvant éventuellement comporter ladite charge minérale, à laquelle on mélange la composition selon l'invention, de sorte que sa concentration en poids dans la dispersion soit telle qu'indiquée ci-dessus.

Une telle dispersion peut comporter de 1 à 10 %, de préférence de 4 à 6 % en poids de matières sèches.

Le mélange de la dispersion et de la composition selon l'invention, entraîne la précipitation des fibres et éventuellement des charges minérales dispersées, auxquelles est associée ladite composition.

Les matières solides peuvent alors être séparées de la phase aqueuse surnageante par un procédé physique de séparation, par exemple par filtration. Puis lesdites matières solides peuvent être mises en forme, séchées et éventuellement cuites. La mise en forme se fait selon une méthode classique, en fonction de l'usage ultérieur de l'article isolant.

Après séchage, ce dernier comporte habituellement de 30 à 90 % en poids de fibres minérales, éventuellement de 5 à 60 % en poids de charges minérales et de 0,5 à 7 %, de préférence de 1 à 5 % en poids, de la composition selon l'invention.

Les articles isolants comportant une composition selon l'invention présentent des propriétés mécaniques accrues par rapport à des articles isolants connus jusqu'alors. Mais surtout, grâce à cette composition, il est possible de réduire notablement la proportion de matières organiques contenue dans ledit article isolant. Or, une quantité trop importante de matière organique présente de nombreux inconvénients, en particulier, l'émission d'une fumée noire lors de la cuisson de l'article isolant.

Au moyen de la composition selon l'invention, il est également possible de fabriquer des articles à base de fibres de bois, notamment selon un tel procédé similaire à celui employé pour fabriquer lesdits articles isolants, décrit ci-dessus. Selon un procédé, lesdites fibres minérales sont remplacées par des fibres de bois.

Les exemples suivants ont pour but d'illustrer la présente invention.

### EXEMPLE

On prépare 100 g de solution de polysaccharides en mélangeant 22 g d'une solution à 0,5% en poids d'un polysaccharide obtenu par fermentation au moyen d'une souche pseudomonas Paucimobilis I-886 avec 22 g d'une solution de guar cationique à 1% en poids et 56 g d'eau potable.

Le polysaccharide obtenu au moyen de ladite souche Pseudomonas Paucimobilis I- 886 ainsi que son procédé de préparation, sont décrits dans la demande de brevet français N° 88 09999.

Le guar cationique utilisé est un produit commercialisé par la société Meyhall sous le nom Meyproid 9806

Dans un bécher de 5 litres, on disperse 22 g de fibres céramiques alumine-silice dans 2100 g d'eau, sous une agitation de 500 tours/min. Puis on introduit dans cette dispersion, toujours sous agitation, la solution de polysaccharides.

Deux minutes après, on arrête l'agitation. Les fibres précipitent. On sépare les fibres précipitées de la solution aqueuse par filtration sous vide sur un Buchner. Le gâteau de filtration est séché.

L'article isolant ainsi obtenu contient 1,5 % en poids de polysaccharides par rapport au poids des fibres.

## Revendications

1. Composition caractérisée en ce qu'elle comporte :
. au moins un polysaccharide (A) dont le motif de base contient du glucose et du rhamnose en quantités relatives correspondant à 1 à 6 moles de glucose pour une mole de rhamnose, ledit polysaccharide étant préparé par fermentation d'un milieu nutritif comportant au moins une source carbonée au moyen d'un microorganisme Alcaligenes ou Pseudomonas;
. ainsi qu'au moins un polysaccharide cationique choisi parmi les dérivés cationiques d'amidon, de galactomannane ou de guar;
selon un rapport poids de polysaccharide (A)/poids de polysaccharide cationique compris entre 5/95 et 95/5.

2. Composition selon la revendication 1 caractérisée en ce que le motif de base dudit polysaccharide (A) contient du glucose et du rhamnose en quantités relatives correspondant à 1 à 4 moles de glucose pour une mole de rhamnose.

3. Composition selon selon la revendication 1 ou 2 caractérisée en ce que le motif de base dudit polysaccharide (A) comporte en outre des restes acétyles ou pyruviles, et/ou de l'acide glucuronique, du galactose, du mannose,de l'arabinose, du fucose ou du ribose.

4. Composition selon l'une quelconque des revendications 1 à 3 caractérisée en ce que ledit microorganisme est Pseudomonas paucimobilis I-886, DSM 4429 ou Alcaligenes ATCC 31961.

5. Composition selon l'une quelconque des revendications 1 à 4 caractérisée en ce que le rapport poids de polysaccharide (A)/poids de polysaccharide cationique est compris entre 30/70 et 70/30.

6. Composition selon l'une des revendications 1 à 5 caractérisée en ce qu'elle comporte en outre un additif floculant.

7. Composition selon la revendication 6 caractérisée en ce que ledit additif floculant est un composé organique ammonium quaternaire, l'ammoniaque, un polymère ou un copolymère organique anionique de synthèse et leurs sels, ou un composé à base d'un métal du groupe du fer et/ou de l'aluminium.

8. Procédé pour précipiter dans un milieu aqueux une dispersion de particules solides caractérisé en ce qu'on mélange ladite dispersion avec une composition selon l'une des revendications 1 à 7.

9. Procédé selon la revendication 8 caractérisé en ce qu'on mélange ladite dispersion avec ladite composition, de sorte que la concentration de la composition dans le milieu aqueux soit comprise entre 0,001 et 0,5 % en poids.

10. Procédé selon la revendication 9 caractérisé en ce que ladite concentration est comprise entre 0,005 et 0,2 % en poids.

11. Procédé selon l'une des revendications 8 à 10 caractérisé en ce que lesdites particules sont des fibres minérales, éventuellement en mélange avec au moins une charge minérale.

12. Procédé selon l'une des revendications 8 à 10 caractérisé en ce que lesdites particules sont des fibres de bois.

13. Articles isolants caractérisés en ce qu'ils comportent une composition selon l'une des revendications 1 à 7, des fibres minérales et éventuellement au moins une charge minérale.

14. Articles isolants caractérisés en ce qu'ils comportent une composition selon l'une des revendications 1 à 7, des fibres de bois et éventuellement au moins une charge minérale.

15. Utilisation de la composition selon l'une des revendications 1 à 7 pour traiter des eaux usées.

## Claims

1. Composition, characterized in that it contains:
. at least one polysaccharide (A), the base unit of which contains glucose and rhamnose in relative amounts corresponding to 1 to 6 mol of glucose per mole of rhamnose, the said polysaccharide being prepared by fermentation of a nutrient medium containing at least one carbon source by means of an Alcaligenes or Pseudomonas microorganism;
. together with at least one cationic polysaccharide chosen from cationic derivatives of starch, of galactomannan or of guar;
according to a weight of polysaccharide (A)/weight of cationic polysaccharide ratio of between 5:95 and 95:5.

2. Composition according to claim 1, characterized in that the base unit of the said polysaccharide (A) contains glucose and rhamnose in relative amounts corresponding to 1 to 4 mol of glucose per mole of rhamnose.

3. Composition according to claim 1 or 2, characterized in that the base unit of the said polysaccharide (A) contains, in addition, acetyl or pyruvoyl residues, and/or glucuronic acid, galactose, mannose, arabinose, fucose or ribose.

4. Composition according to any one of claims 1 to 3, characterized in that the said microorganism is Pseudomonas paucimobilis I-886 or DSM 4429 or Alcaligenes ATCC 31961.

5. Composition according to any one of claims 1 to 4, characterized in that the weight of polysaccharide (A)/weight of cationic polysaccharide ratio is between 30:70 and 70:30.

6. Composition according to one of claims 1 to 5, characterized in that it contains, in addition, a flocculating additive.

7. Composition according to claim 6, characterized in that the said flocculating additive is an organic quaternary ammonium compound, aqueous ammonia, a synthetic anionic organic polymer or copolymer and their salts, or a compound based on a metal of the iron and/or aluminium group.

8. Process for precipitating a dispersion of solid particles in an aqueous medium, characterized in that the said dispersion is mixed with a composition according to one of claims 1 to 7.

9. Process according to claim 8, characterized in that the said dispersion is mixed with the said composition so that the concentration of the composition in the aqueous medium is between 0.001 and 0.5% by weight.

10. Process according to claim 9, characterized in that the said concentration is between 0.005 and 0.2% by weight.

11. Process according to one of claims 8 to 10, characterized in that the said particles are inorganic fibres, optionally mixed with at least one inorganic filler.

12. Process according to one of claims 8 to 10, characterized in that the said particles are wood fibres.

13. Insulating articles, characterized in that they contain a composition according to one of claims 1 to 7, inorganic fibres and optionally at least one inorganic filler.

14. Insulating articles, characterized in that they contain a composition according to one of claims 1 to 7, wood fibres and optionally at least one inorganic filler.

15. Use of the composition according to one of claims 1 to 7, for treating waste water.

## Patentansprüche

1. Zusammensetzung, dadurch gekennzeichnet, daß sie enthält:
- wenigstens ein Polysaccharid (A), dessen Grundbaustein Glucose und Rhamnose enthält, und zwar in relativen Mengen, die 1 bis 6 Molen Glucose auf ein Mol Rhamnose entsprechen, wobei besagtes Polysaccharid durch Fermentation eines Nährmediums hergestellt ist, das zumindest eine Kohlenstoffquelle enthält, mit Hilfe eines Mikroorganismus der Gattung Alcaligenes oder Pseudomonas;
- sowie wenigstens ein kationisches Polysaccharid, ausgewählt aus den kationischen Stärke-, Galactomannan- oder Guarderivaten;
bei einem Verhältnis Gewicht des Polysaccharids (A)/Gewicht des kationischen Polysaccharids von zwischen 5/95 und 95/5.

2. Zusammensetzung nach Anspruch 1, dadurch gekennzeichnet, daß der Grundbaustein besagten Polysaccharids (A) Glucose und Rhamnose enthält, und zwar in relativen Mengen, die 1 bis 4 Molen Glucose auf ein Mol Rhamnose entsprechen.

3. Zusammensetzung nach Anspruch 1 oder 2, dadurch gekennzeichnet, daß der Grundbaustein besagten Polysaccharids (A) außerdem Acetyl- oder Pyruvilreste enthält und/oder Glucuronsäure, Galactose, Mannose, Arabinose, Fucose oder Ribose.

4. Zusammensetzung nach einem beliebigen der Ansprüche 1 bis 3, dadurch gekennzeichnet, daß besagter Mikroorganismus Pseudomonas paucimobilis I-886, DSM 4429 oder Alcaligenes ATCC 31961 ist.

5. Zusammensetzung nach einem beliebigen der Ansprüche 1 bis 4, dadurch gekennzeichnet, daß das Verhältnis Gewicht des Polysaccharids (A)/Gewicht des kationischen Polysaccharids zwischen 30/70 und 70/30 liegt.

6. Zusammensetzung nach einem der Ansprüche 1 bis 5, dadurch gekennzeichnet, daß sie außerdem ein Flockungsmittel enthält.

7. Zusammensetzung nach Anspruch 6, dadurch gekennzeichnet, daß besagtes Flockungsmittel folgende Verbindung ist: eine organische quartäre Ammoniumverbindung, Ammoniak, ein organisches anionisches synthetisches Polymer oder Copolymer und ihre Salze oder eine Verbindung, die auf einem Metall der Eisengruppe und/oder der Aluminiumgruppe basiert.

8. Verfahren zur Ausfällung einer Dispersion fester Partikel aus einem wäßrigen Medium, dadurch gekennzeichnet, daß man besagte Dispersion mit einer Zusammensetzung nach einem der Ansprüche 1 bis 7 vermischt.

9. Verfahren nach Anspruch 8, dadurch gekennzeichnet, daß man besagte Dispersion mit besagter Zusammensetzung mischt, so daß die Konzentration der Zusammensetzung in dem wäßrigen Medium zwischen 0,001 und 0,5 Gewichtsprozent liegt.

10. Verfahren nach Anspruch 9, dadurch gekennzeichnet, daß besagte Konzentration zwischen 0,005 und 0,2 Gewichtsprozent liegt.

11. Verfahren nach einem der Ansprüche 8 bis 10, dadurch gekennzeichnet, daß besagte Partikel Mineralfasern sind, gegebenenfalls vermischt mit zumindest einem mineralischen Füllstoff.

12. Verfahren nach einem der Ansprüche 8 bis 10, dadurch gekennzeichnet, daß besagte Partikel Holzfasern sind.

13. Isolierartikel, dadurch gekennzeichnet, daß sie eine Zusammensetzung nach einem der Ansprüche 1 bis 7, Mineralfasern und gegebenenfalls wenigstens einen mineralischen Füllstoff enthalten.

14. Isolierartikel, dadurch gekennzeichnet, daß sie eine Zusammensetzung nach einem der Ansprüche 1 bis 7, Holzfasern und gegebenenfalls wenigstens einen mineralischen Füllstoff enthalten.

15. Verwendung der Zusammensetzung nach einem der Ansprüche 1 bis 7 zur Behandlung von Abwässern.
